# EUROPEAN PATENT SPECIFICATION

(11) **EP 3 128 909 B1**
(45) Date of publication and mention of the grant of the patent: **10.11.2021**
(21) Application number: 15776448.1
(22) Date of filing: 10.04.2015
(51) Int. Cl.: A61B 5/06, A61B 8/13

(54) **IMPLANT DELIVERY SYSTEM**
IMPLANTATEINFÜHRUNGSSYSTEM
SYSTÈMES DE POSE D'IMPLANT

(30) Priority: 11.04.2014 US 201414250634
(43) Date of publication of application: 15.02.2017
(73) Proprietor: Koninklijke Philips N.V., 5656 AG Eindhoven (NL)
(72) Inventor: LEBLANC, Christopher, San Diego, CA 92128 (US); STIGALL, Jeremy, Carlsbad, CA 92009 (US)
(74) Representative: Philips Intellectual Property & Standards
(86) International application number: PCT/US2015/025438
(87) International publication number: WO 2015/157709

(56) References cited:
- WO-A1-2014/100382
- US-A- 5 156 154
- US-A1- 2007 239 024
- US-A1- 2008 009 746
- US-A1- 2008 119 879
- US-A1- 2009 234 445
- US-A1- 2013 253 309
- US-A1- 2013 253 346
- US-A1- 2013 267 848
- US-A1- 2013 338 752
- US-A1- 2014 005 529
- US-A1- 2014 180 067
- None

## Description

### Technical Field

The present invention generally relates to system for guided delivery of replacement heart valves, such as valve stents, into the vasculature.

### Background

Heart valves help regulate blood flow through the heart chambers, the left and right atria and the left and right ventricles, each of which includes its own one-way exit valve. These valves are identified as the aortic, mitral (or bicuspid), tricuspid and pulmonary valves. When functioning normally, the valves maintain one-way blood flow through the heart during each heartbeat and prevent backflow.

When a valve malfunctions or is diseased, it may constrict the volume of blood allowed through the valve or permit blood to flow back within the heart. Constriction (known as stenosis) is the abnormal narrowing of a valve due to, e.g., a build of calcium deposits, tissue, or scarring. Backflow (known as insufficiency or regurgitation) occurs when the valve structure is torn, loose, stretched, or thin. Several factors may cause valve stenosis and/or insufficiency such as congenital valve disease, infection, and injury.

Constriction and/or backflow require the heart to work harder in order to supply adequate blood throughout the body. Left untreated, abnormal blood flow associated with malfunctioning or diseased valves can have serious side effects, such as shortness of breath, heart attack, stroke, and even death. In light of the serious side effects, heart valve replacement surgery is often indicated to correct the disruption of blood flow from the heart.

Heart valve replacement surgery introduces a valve implant (i.e. valve stent) to replace the dysfunctional native valve. Conventional heart valve replacement surgery is a highly invasive, open-heart procedure conducted through the patient's sternum. More recently, percutaneous heart valve replacement procedures have emerged as a safer, less-invasive alternative to open-heart surgery. These less-invasive procedures typically involve introducing a delivery catheter under fluoroscopic guidance to deliver a replacement valve implant. While less invasive, the fluoroscopic guidance alone prevents precise placement of the implant with the catheter and does not provide intraluminal assessment of the valve before, during, and after the valve replacement surgery.

US 2014/0005529 A1 describes a combined multi-lumen central access catheter and an embolic filter, further comprising an imaging modality to facilitate intravascular imaging of the filter. The multi-lumen catheter with associated filter member serves as the sheath for introduction of an intravascular imaging system, permitting improved imaging of the condition of the filter member while in use. In particular, the use of an intravascular imaging system with the multi-lumen catheter with associated filter member will permit a medical professional to monitor the condition of the filter member, and utilize appropriate techniques to lyse a collected thrombus.

US 2013/0267848 A1 relates to percutaneously delivering or retrieving vascular implant devices, such as filters, utilizing intravenous ultrasound (IVUS) imaging alone or in combination with external (e.g. transabdominal) ultrasound or other imaging technology. Implants deliverable by such systems, such as vena cava or other vascular filters, can have two or more echogenic markers spaced at such a distance that they are separately discernible by IVUS and/or external ultrasound imaging.

US 2008/0119879 A1 relates to the formation of an arteriovenous fistula in the limb of the patient. Embodiments include a device for the creation, modification and maintenance of a fistula that includes an integral fistula creation assembly near its distal end that passes through the skin of the patient, through a first vessel such as an artery, and into a second vessel such as a vein. The fistula creation assembly preferably includes an anastomotic implant that is placed within the fistula to maintain long-term blood flow therethrough. The devices, systems and methods can be used to treat patients with one or more numerous ailments including chronic obstructive pulmonary disease, congestive heart failure, hypertension, hypotension, respiratory failure, pulmonary arterial hypertension, lung fibrosis and adult respiratory distress syndrome.

US 2008/0009746 A1 relates to assessing the size, shape, and topography of vessel lumens and hollow portions of organs. The devices and methods are particularly adapted for determining the size, shape, topography, and compliance of the native heart valves to facilitate the later implantation of a prosthetic heart valve. The devices are typically catheter-based having an assessment mechanism fixed to a distal end of the catheter. The assessment mechanism generally includes an expandable member, such as a balloon. The assessment mechanism may also include an imaging member, a physical assessment member, an electronic mapping construction, an alignment mechanism, a valvuloplasty balloon, or any combinations thereof. The methods typically comprise deploying the balloon percutaneously to a target location, expanding the balloon, and determining one or more physical parameters associated with the target location.

### Summary

The object of the present invention is solved by the subject-matter of the independent claim; further embodiments are incorporated in the dependent claims.

The present invention utilizes a single catheter device with intravascular imaging and functional flow sensing capabilities to guide and facilitate valve replacement surgery. Systems of the invention can be used to verify a location of a diseased valve, assess the condition of the diseased valve, visualize implantation of the replacement valve within the heart, assess placement of the deployed replacement valve, and evaluate restoration of blood flow after replacement. With the enhanced guidance provided by the present catheter systems, the risk of unnecessarily injuring the heart or surrounding vasculature tissue due to improper deployment/placement of a valve stent or other implant is minimized. Because systems of the invention require only one catheter for pre-implantation assessment, valve implantation, and post-implantation assessment, the number of catheters that are introduced into the vasculature is minimized. This reduces the risk associated with exchanging and moving multiple catheters within the delicate vasculature that may already be weakened by disease.

Aspects of the invention are accomplished with a system for delivering an implant, wherein the system comprises a device for delivering an implant (such as a valve stent) that includes an imaging element and functional flow sensors. The delivery device includes an outer sheath defining a center lumen that leads to a distal opening. An implant can be releasably held within the center lumen or driven through the center lumen. The outer sheath also includes an imaging element configured to at least partially surround the center lumen such that the implant is deployable through the imaging element, wherein the imaging element is positioned on or formed as part of an outer surface of a distal end of the outer sheath. Because the implant passes through the imaging element and then deploys into the body lumen, the imaging element does interfere with implant deployment. Moreover, this configuration provides a small profile because the implant and imaging element are concentrically aligned. The imaging element can be used to obtain intraluminal images of the vasculature to assess the condition of the diseased valve, determine the appropriate placement of the catheter for valve delivery, and assess placement of the valve stent as deployed within the heart.

For deployment of the implant, the delivery device of the invention includes an inner member disposed within the outer sheath's center lumen such that movement of the inner member relative to the outer sheath (or vice versa) deploys an implant from the distal opening of the outer sheath. The inner member may be a push rod or an inner catheter sheath. In certain embodiments, the inner member is used to push the implant so that the implant deploys out of the center lumen. Alternatively, the inner member can be used to maintain the positioning of the implant relative to the outer sheath (or elongate body) such that proximal movement of the outer sheath deploys the implant out of the device and into the implantation site.

The implant delivery device also includes sensors in combination with the imaging element. The sensors are used to obtain functional flow measurements, and include a pressure sensor and a flow sensor. A benefit of pressure/flow sensors is that one is able to obtain functional flow measurements of the vasculature. Functional flow measurements can be used to assess the health of the valve and circulation prior to implantation and/or assess blood flow and circulation after placement of the implant in order to confirm proper deployment. The sensors are located near a distal end of the implant delivery device, and in close proximity to the opening through which the implant is deployed and are positioned on or formed as part of the outer surface of the outer sheath.

Moreover, the delivery device comprises a connector fitting attached to a proximal portion of the device.

Further, the system comprises an operating system providing a means to transmit electricity and receive data from the imaging elements and the pressure and flow sensors of the delivery device, wherein through the connector fitting and via a cable the imaging element and the pressure and flow sensors are operably coupled to the operating system. The system is configured to compare the measured pressure and velocity of flow to determine an index of resistance of flow within the vasculature.

The outer catheter sheath (i.e. elongate body) of the delivery system may include one or more radiopaque markers along the length of the catheter sheath. The radiopaque markers allow one to determine the positioning of the catheter relative to the vasculature when viewed with an external imaging modality (such as fluoroscopy). This further aids in determining whether the catheter is appropriately placed for implantation of, e.g., a replacement valve.

Certain aspects of the invention provide that the inner member (e.g. push rod or inner sheath) includes a second imaging element. After the implant is deployed into a lumen (such as a heart valve) and engaged with the luminal wall, the inner member may enter the lumen and its imaging element may be used to image the implant as implanted. A particular benefit of this aspect is that the second imaging element can image the implant as engaged with the luminal wall without touching or interfering with the implant because the inner member has a smaller profile than the elongate body and is able to fit within a cavity formed by the deployed implant (such as a valve or a filter). For example, if the implant is filter, the filter legs expand from a center point and engage with the vessel walls, thereby creating a funnel-like cavity between the filter legs. The inner member may move within the funnel-like cavity between the expanded wire legs to image the legs as engaged with the vessel wall.

Systems of the present invention may be used in a variety of body lumens, including but not limited to intravascular lumens such as heart chambers and coronary arteries. Typically, devices of the invention are used to a) deploy valve implants to replace diseased/damaged native valves of the heart or b) to deploy filter implants within the vasculature to prevent thrombi from travelling within the bloodstream. However, devices of the invention can be used to delivery other implants for a variety of reasons. For example, the implant may be introduced into a vessel to occlude the vessel downstream and eliminate blood flow within the vessel. In another example, the implant may be introduced into a vessel to provide open mechanical support to a diseased vessel. Suitable implants for delivery into a body lumen include, but are not limited to a plug, a stent, a pH sensor, a pressure monitor, a plug, a filter, or a valve. The implant may be expandable, such as self-expandable valve stents or filters.

In certain aspect, an implant delivery device of the invention includes an outer catheter sheath and an inner catheter sheath. The outer catheter sheath includes a first imaging element and defines a center lumen that leads to an opening. The outer catheter sheath is configured to releasably hold an implant within the center lumen or receive the inner catheter sheath engaged with an implant within the center lumen. The first imaging element is configured to at least partially surround the center lumen such that the implant is deployable through the first imaging element. The inner catheter sheath is moveably disposable within the center lumen of the outer catheter sheath. Movement of the inner catheter sheath or outer catheter sheath relative to each other deploys the implant into a desired implantation site.

The first imaging element may be positioned on or formed as part of an outer surface of a distal end of the outer catheter sheath. In certain embodiments, the first imaging element surrounds the distal end of the outer catheter sheath at a position slightly proximal (e.g. two millimeters or less) to the opening of center lumen. With this arrangement, the operator is able to locate an implantation site with the first imaging element, and then position outer catheter sheath for implant deployment such that the opening is slightly proximal to the located implantation site. In this manner, the operator can know that the implant is being delivered at a location slightly distal to real-time images being obtained from the first imaging element.

In order to facilitate deployment of the implant out of the center lumen and into a body lumen, the inner catheter sheath can include a push element configured to engage with the implant. For example, the push element of the inner catheter sheath may include a substantially flat surface that is substantially flush with the wall of the center lumen. The push element may form the distal end of the inner catheter sheath. In certain embodiments, the inner catheter sheath further includes a second imaging element. The second imaging element may be proximal to the push element of the inner catheter sheath. The second imaging element can be used to obtain images of the luminal surface when at least a portion of the inner catheter sheath is deployed out of the opening of the center lumen. In this manner, the second imaging element allows one to obtain images of the implant as implanted within the body lumen.

The first and second imaging elements can be a component of any known intraluminal imaging apparatus. Suitable imaging apparatus for use with the implant delivery device of the invention include, for example, optical-acoustic sensor apparatuses, intravascular ultrasound (IVUS) or optical coherence tomography (OCT).

Other and further aspects and features of the invention will be evidence from the following detailed description and accompanying drawings, which are intended to illustrate, not limit, the invention. The invention is defined by the independent claim, methods are not part of the invention. Only systems wherein the outer sheath comprises a pressure sensor adapted to measure pressure within the vasculature and a flow sensor adapted to measure the velocity of flow through the vasculature are part of the invention. Only systems wherein the pressure and flow sensors are located on a distal portion of the outer sheath distal or proximal to the imaging element and are positioned on or formed as part of the outer surface of the outer sheath are part of the invention.

### Brief Description of the Drawings

FIG. 1 depicts a distal end of an implant delivery device according to one embodiment.
FIG. 2 shows an implant delivery device according to certain embodiments.
FIG. 3 depicts a longitudinal cross-section of a distal portion of the delivery device according to certain embodiments.
FIG. 4 depicts another cross-section along the x-axis of a distal portion of the delivery device according to certain embodiments.
FIG. 5 depicts an alternative embodiment of the delivery device.
FIGS. 6-11 illustrate an exemplary delivery device of the invention in operation.
FIG. 12A-12C depicts filters according to certain embodiments.
FIG. 13 is a system diagram according to certain embodiments.
FIGS. 14A-C depict an alternative embodiment of the delivery device.

### Detailed Description

The present invention generally relates to intraluminal systems with combined implant delivery and imaging capabilities. The delivery systems of the invention provide for 1) real-time imaging of intraluminal surfaces to detect/evaluate a location of interest (e.g. implantation site); 2) real-time monitoring of functional flow parameters (e.g. pressure/flow) within the vasculature to detect/evaluate the location of interest; 3) delivery of an implant into the implantation site; 4) real-time imaging of the implant as engaged with the intraluminal surface without interfering with the implant as implanted; and 5) real-time monitoring of functional flow parameters within the vasculature after implantation to evaluate blood flow and circulation.

Because the above features are accomplished with one system introduced into a body lumen, this present invention eliminates the need to introduce multiple catheters into the body. For example, there is no need to introduce and remove an imaging or sensor catheter to locate a region of interest, then introduce and remove a delivery catheter to deliver an implant, and then re-introduce the imaging or sensor catheter to evaluate the implant as implanted.

In certain embodiments, systems of the invention image and deliver an implant within a lumen of tissue. Various lumen of biological structures may be imaged and receive an implant including, but not limited to, blood vessels, vasculature of the lymphatic and nervous systems, various structures of the gastrointestinal tract including lumen of the small intestine, large intestine, stomach, esophagus, colon, pancreatic duct, bile duct, hepatic duct, lumen of the reproductive tract including the vas deferens, uterus and fallopian tubes, structures of the urinary tract including urinary collecting ducts, renal tubules, ureter, and bladder, and structures of the head and neck and pulmonary system including sinuses, parotid, trachea, bronchi, and lungs.

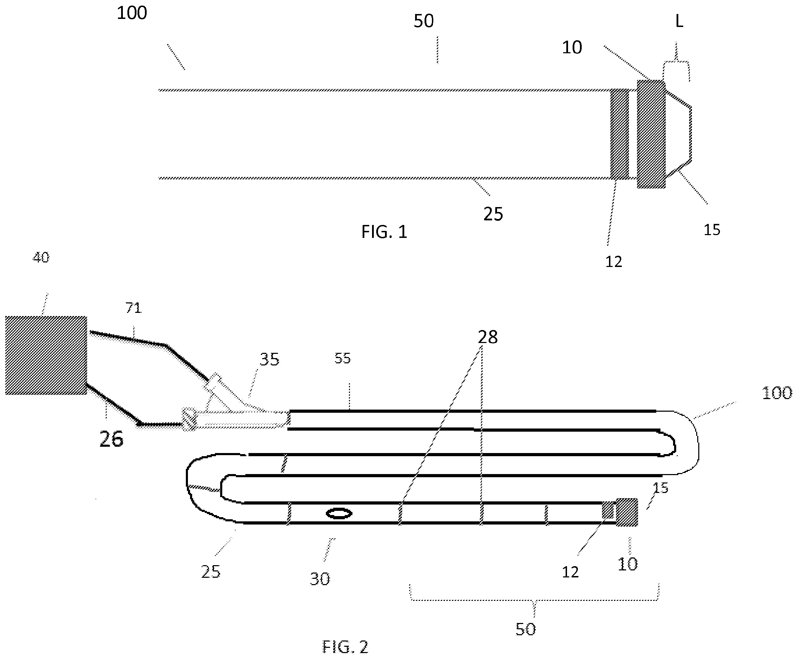

The delivery system may be used to deliver any suitable implant into a body lumen. Suitable implants for delivery into the lumen include a stent, a plug, a pH sensor, pressure monitor, a plug, a filter, or a valve. The delivery system may be used to deliver an implant for a variety of reasons. For example, the implant may be introduced into a vessel to occlude the vessel downstream to eliminate blood flow within the vessel. In another example, the implant may be introduced into a vessel to provide open mechanical support to a diseased vessel. In yet another example, delivery devices are well suited to deliver a replacement valve (such as a valve stent) into the heart to replace a damaged or diseased valve. The implant used for purposes of describing the components and function of the delivery system depicted in FIGS. 1-11 is a filter.

In particular embodiments and as discussed in reference to FIGS. 1-11, the delivery system of the invention can be used to deploy a filter into vessel of the vasculature to block thrombi from traveling through the blood stream. (Although it is understood that other implants (such as valve stents) can be delivered in the same or similar manner.) These types of filters are typically introduced into the inferior vena cava vein to block thrombi originating in the lower extremities from breaking off and traveling through the bloodstream. Prior to filter placement, the surgeon must take care to locate the proper filter location. The optimal location for filter placement is in the infrarenal inferior vena cava with the apex of the filter just below the level of the lowest renal vein because, at this level, a thrombus caught by the filter will be exposed to renal vein blood flow, which may promote dissolution by the intrinsic lytic system. In order to determine appropriate placement of a vena cava filter, a surgeon must take care to avoid encroachment on the renal veins and to ascertain the absence or presence of thrombus within the vena cava. For example, a filter placed at or above the renal veins can lead to renal vein thrombosis and deterioration of renal function if the filter, and thus the vessel, become occluded. In addition, the filter should not be placed on a thrombus tissue, but rather should be placed on healthy vessel walls to ensure the filter engages with the wall of the vena cava.

The delivery system of the invention may optionally involve the introduction of an introducer sheath. Introducer sheaths are known in the art. Introducer sheaths are advanced over the guidewire into the vessel. A catheter or other device may then be advanced through a lumen of the introducer sheath and over the guidewire into a position for performing a medical procedure. Thus, the introducer sheath may facilitate introducing the catheter into the vessel, while minimizing trauma to the vessel wall and/or minimizing blood loss during a procedure.

FIG. 1 depicts the distal portion 50 of a delivery system 100 according to certain embodiments. The delivery system 100 includes an elongate body 25 and an imaging element 10 located near a distal tip 15 of the elongate body 25. An opening (not shown in FIG. 1) is open in the distal direction on the distal tip 15, through which an implant can be delivered into a body lumen. The imaging element 10 is a distance L from the distal tip 15. In certain embodiments, the imaging element is positioned 2 millimeters or less from the distal tip 15. Ideally, the imaging element 10 is positioned as close as possible to the distal tip 15 to minimize the distance between the actual location of the imaging and an implant delivery site. The imaging element 10 can partially or fully surround the elongate body 25. In addition, the imaging element 10 can be positioned on or formed as part of an outer surface of the distal end 50 of the elongate body 25. In preferred embodiments, the imaging element 10 surrounds the elongate body 25 to provide cross-sectional imaging of the body lumen (i.e. to provide a 360 degree slice of the vessel at different longitudinal sections of the body lumen). If the imaging element 10 only partially surrounds the elongate body 25, the elongate body 25 could be configured to rotate to provide cross-sectional imaging.

In all aspects, delivery systems 100 of the invention include more sensor elements 12 in combination with one or more imaging elements 10. The one or more sensor elements 12 may include a pressure sensor, flow sensor, temperature sensor, or any combination thereof. Sensor elements 12 and methods of using information obtained from sensor elements 12 are described in more detail hereinafter.

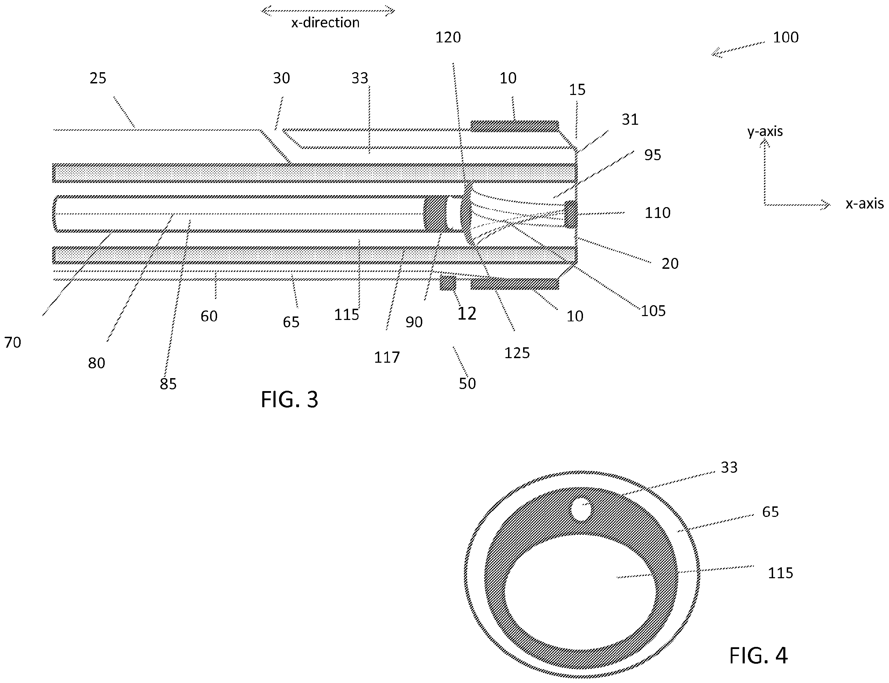

FIG. 2 further depicts a delivery device 100 of the invention according to certain embodiments. The delivery device 100 is shown coupled to a connector. Connector fitting 35 is attached at a proximal portion 55 of the elongate body 25. Connector fitting 35 provides a functional access port at the proximal end of devices of the invention. For example, an inner member 70 (not shown in FIG. 2) or delivery sheath can be telescoped through the elongate body 25 through the connector fitting 35. Through the connector fitting 35 and via cable 26, the imaging element 10 and/or one or more sensors 12 elongate body 25 are operably coupled to an operating system 40. In addition, a proximal end 71 of the inner member 70 be coupled to the operating system 40. (The inner member 70 is shown within the center lumen of the elongate body in FIG. 3.) It is also understood that cable 26 and proximal end 71 can be routed through either port of the connector fitting 35. Typically, the operating system 40 provides a means to transmit electricity and receive data from imaging elements 10 and sensors 12 of the delivery systems. The operating system 40 can be a component of computerized ultrasound assembly equipment, optical coherence tomography assembly equipment, functional flow equipment or equipment of another imaging/sensor system. Various imaging assemblies and sensor assemblies suitable for use with devices of the invention are described in more detail hereinafter.

In addition, the operating system 40 can be configured to provide a controlled translation of the inner member with respect to the elongate body 25. As alternative to one operating system, the elongate body 25 and the inner member 70 can be coupled to separate operating systems.

Referring back to FIG. 2, the elongate body 25 of the delivery device 100 may further include one or more radiopaque markers 28 located along the length of the elongate body 25. The radiopaque markers 28, when used in conjunction with an external imaging modality such as an angiogram, indicate the positioning of the catheter as disposed within a subject. The radiopaque markers 28 may be formed from a radiopaque object or a radiopaque ink. In certain embodiments, the radiopaque markers 28 are spaced a certain distance apart in order to allow a quick assessment as to the relative position of the elongate body 25 within the vasculature. The spatially-separated radiopaque markers 28 may indicate when the device is a certain distance within a particular vessel of interest. For example, the radiopaque markers 28 may indicate when the device is 25 cm inside the femoral vein, which is an ideal distance within the vein to place an implant such as a valve stent.

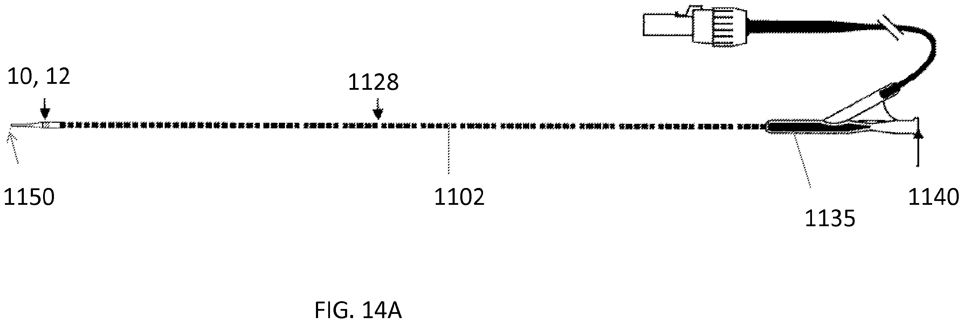

FIG. 2 also shows a guidewire opening 30 near the distal portion 50 of the elongate body 25. As shown in FIG. 2, the delivery system 100 is a rapid exchange catheter device. However, the delivery system 100 can be designed as an over-the-wire system or a rapid exchange system. Over-the-wire catheters include a guidewire lumen that runs the full length of the catheter (and is often the same as a catheter's center lumen). Rapid exchange catheters include a guidewire lumen extending only through a distal portion of the catheter. With respect to the remaining proximal portion of the catheter, the guidewire exits the internal catheter lumen through a guidewire opening 30, and the guidewire extends in parallel along the proximal catheter portion. Delivery systems of the invention that include an over-the-wire configuration are depicted in FIGS. 14A-14C.

FIG. 3 depicts a cross-section of the distal portion 50 of the delivery device 100 shown in FIG. 1 according to certain embodiments. The delivery device 100 includes an elongate body 25 that defines a center lumen 115. The center lumen 115 extends to an opening 20 through which an implant can be deployed. The elongate body 25 includes an imaging element 10 positioned on or formed as part of the outer surface of the elongate body 25. In addition, the elongate body 25 includes one or more sensors 12 positioned on or formed as part of the outer surface of the elongate body 25. The imaging element 10 is a component of an imaging assembly, and the one or more sensors 12 may be a component of a sensor assembly, which are described in more detail hereinafter. The imaging element 10 and sensor 12 of the elongate body can be connected to transmission line(s) 60. The transmission line(s) 60 are configured to transmit electricity to the imaging element 10 and sensors 12 and receive imaging/sensor signals from the imaging element 10 and sensors 12. The transmission(s) line 60 is disposed through a transmission lumen 65 of the elongate body. The transmission line(s) 60 can include one or more signal lines, e.g. one or more signal lines specific to the imaging element 10 and one or more single lines specific to the sensor 12.

An inner member 70 is disposed within the center lumen 115 of elongate body 25. The inner member 70 is movable within the center lumen 115 and can translate with respect to the elongate body 25 in the forward (distal) and backward (proximal) directions, as indicated by arrow x. In addition, the elongate body 25 can translate and move relative to the inner member 70. In certain embodiments, the inner member 70 is moved distally within the center lumen 115 to engage the inner member 70 with a filter 95 and to push the filter 95 into a body lumen. The inner member 70 may be a push rod or an inner catheter sheath.

According to certain embodiments, the inner member 70 includes a push member 120 located at a distal end of the inner member 70. The push member 120 engages with a proximal end of the filter 95 (or other implant). As shown in FIG. 3, the push member 120 engages with the hooked ends 125 of the filter legs 105. The push member 120 can be a flat or slightly-cupped shaped surface (as shown) and can extend the width of the center lumen. The slightly-cupped shaped surface of the push member 120 acts to contain the legs and minimize the hooks ends 125 from engaging with the surface 117 of the center lumen 115. In addition, the push-member can be shaped to specially mate with the filter being deployed. In certain embodiments, the surface 117 of the center lumen 115 that is exposed to the filter hooks ends 125 is formed of a material that prevents the hook ends 125 from perforating, penetrating, or catching on the surface 117 of the center lumen 115 during deployment of the filter 95.

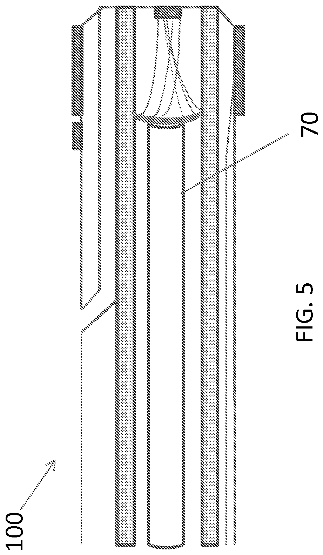

In certain embodiments and as shown, the inner member 70 includes a center member imaging element 90. The center member imaging element 90 is a component of an imaging assembly, which are described in more detail hereinafter. The center member imaging element 90 is proximal to the push member 120. Preferably, the distance between the center member imaging element 90 and the push member 120 is minimized so that the imaging element 90 substantially images from a distal end of the inner member 70. Like the imaging element 10 of the elongate body 25, the imaging element 90 of the inner member 70 can surround the inner member to provide for cross-sectional imaging (360 degree) of the body lumen. If the center member imaging element 90 only partially surrounds the inner member 70, the inner member 70 could be configured to rotate to provide cross-sectional imaging. The center member imaging element 90 is connected to a transmission line 80, which transmits electricity to the center member imaging element 90 and receives imaging signals from the imaging element 90. The transmission line 80 can include one or more signal lines. The transmission line 80 can be disposed within a lumen 85 of the inner member 70. Alternatively, the transmission line 80 can be integrated into the body of the inner member 70. FIG. 5 depicts an alternative embodiment of the delivery device in which the inner member 70 does not include a center member imaging element.

The elongate body 25 further includes a rapid exchange guidewire lumen 33. The guidewire lumen has a guidewire opening 30 at a proximal end and a guidewire opening 31 at a distal end. The elongate body 25 can be guided over a guidewire (not shown) extending through the guidewire lumen 33. During implant deployment, the guidewire can be retracted into the guidewire lumen 33 to prevent the guidewire from interfering with the implant 95 as it is being deployment. Alternatively, the delivery device 100 can be configured as an over-the-wire device.

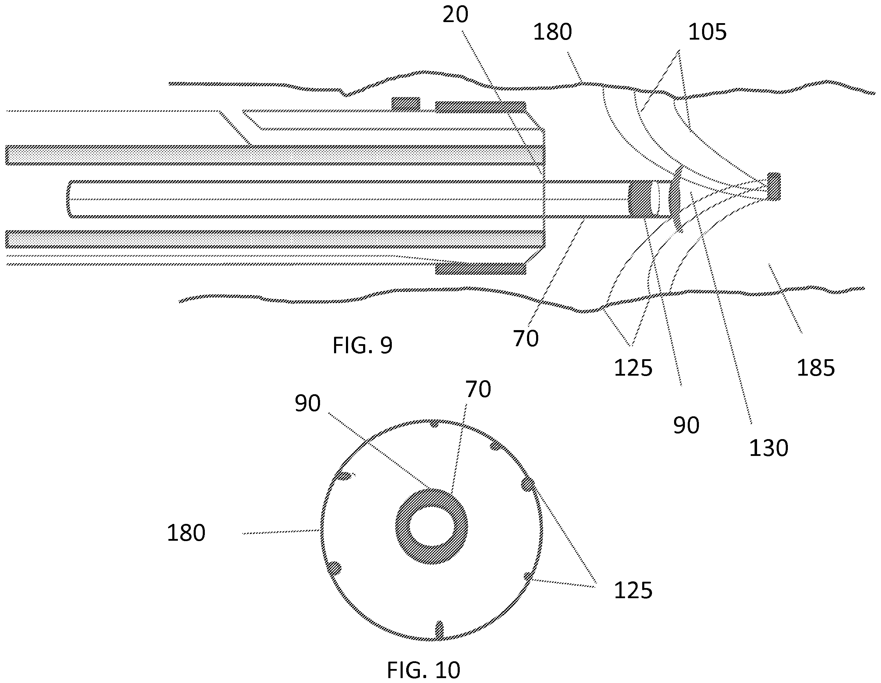

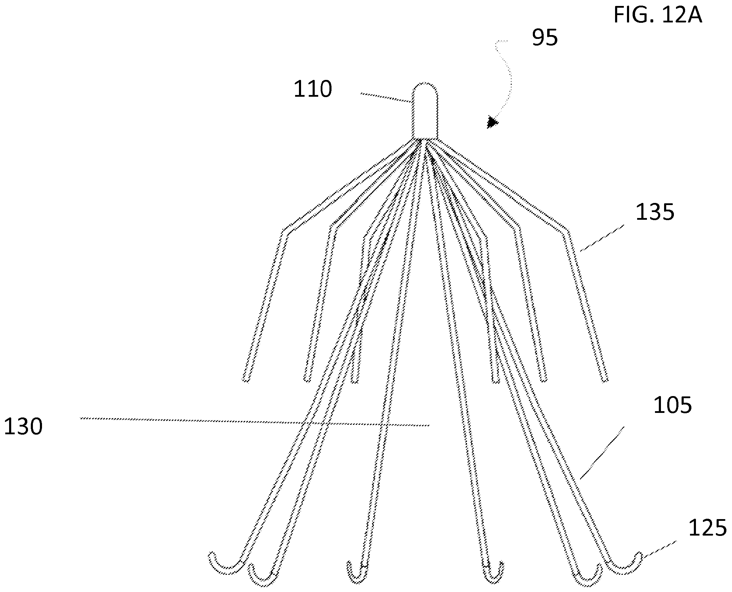

The elongate body 25 is also configured to releasably hold an implant, such as the filter 95 as shown. The filter 95 includes a plurality of legs 105 connected to a center hub 110. The filter legs 105 are configured to expand radially to engage with a surface of a body lumen when fully deployed. As shown in FIG. 3, the filter legs 105 are in a contracted state. FIGS. 9 and 12a depict a filter 95 in its fully expanded state. The filter 95 includes a plurality of legs 150 with hook ends 125. The hook ends 125 secure the filter 95 into the body lumen. The plurality of legs 105 forms a funnel-like cavity 130 between the legs 105. As discussed more fully hereinafter, the inner member 70 can move within the funnel-like cavity 130 of an implanted filter 95 to obtain images of the filter as implanted. In addition and as shown in FIG. 12A, a filter 95 can include capture members 135 that act to further prevent a thrombus from passing through the filter. In addition to the filter 95 shown, most commercially available filters can be used with the delivery device of the invention. Suitable filters are described in U.S. Patent Nos. 6468290, 7534251, and 7972353.

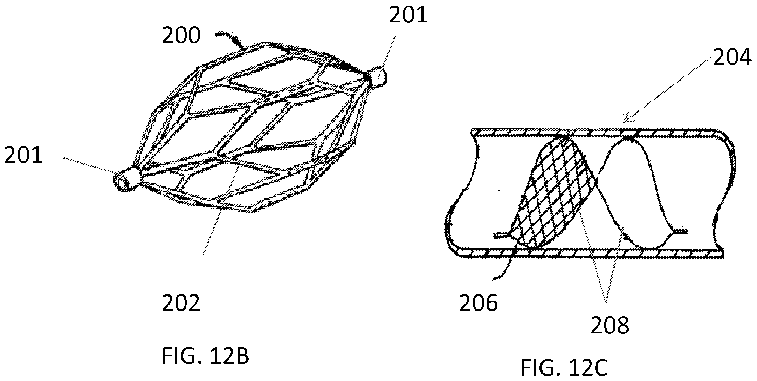

FIGS. 12B-12C illustrate other filters suitable for use in devices and methods of the invention. FIG. 12B illustrates a caged filter 200 that includes a mesh, collapsible cage body disposed between end portions 201. The end portions 201 define a lumen so that the cage filter may be ridden over a guidewire. FIG. 12C depicts a winged-filter 208 disposed within a vessel. The winged-filter 208 includes an expandable two-part frame with a mesh portion 206 disposed within at least one part of the frame.

Each of the filters illustrated in FIGS. 12A-12C are configured to expand to securely-fit against the vessel wall. This allows the filters to capture/snare blood clots traveling through the vasculature without risk of dislocating the filters.

Typically, a filter implanted within a blood vessel is retrieved from the vessel after a certain period of time. There are often several issues associated with the retrieval that can lead to damage to the patient's vessel or result in leaving the filter within the patient permanently, which could deteriorate the vessel wall. In order to overcome the issues associated with retrieval of filters and filters left in the body permanently, certain embodiments of the invention provide filters with bioabsorable properties that decay over time and eventually absorb into the body's blood stream and/or tissue. A filter of the invention may be made with any material having bioabsorable properties, such as magnesium alloys and bioabsorbable polymers. Specifically, bioabsorbable material may include, for example, magnesium alloys, polyglycolic acid, polygalctin 910, poliglecaprone, polydioxanone, poly-α-hydroxy acids, e.g. polylactides, polyglycolides and their copolymers, polyanhydrides, polyorthoesters, segmented block copolymers of polyethylene glycol and poly terephthalate, tyrosine derivative polymers or poly(ester amides). Filters having one or more components may be formed from the same or different bioabsorable materials.

In certain embodiments, the elongate body can include radiopaque markers on the distal tip 15 and the imaging element 10 to assist in determining the location of the elongate body in the vasculature relative to the images obtained by the imaging element. This will allow an operator to visualize the location of the delivery device within the vasculature via an angiogram. The imaging obtained by imaging element 10 may be co-located with the radiopaque markers as described in co-assigned and co-pending application entitled, "LOCATING INTRAVASCULAR IMAGES".

FIG. 4 depicts a cross-section along the y-axis of the elongate body 25 shown in FIG. 3. FIG. 4 illustrates the lumens of elongate body 25. As discussed, the elongate body 25 includes center lumen 115, transmission lumen 65, and a guidewire lumen 33. The transmission lumen 65 does not have to fully surround a portion of the elongate body 25.

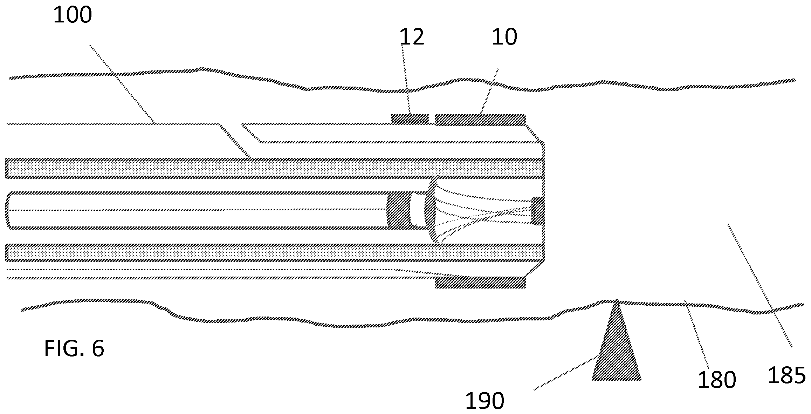

FIGS. 6-11 illustrate the delivery device illustrated in FIG. 3 in operation. FIG. 6 shows the delivery device 100 disposed within a lumen 185 of vessel 180. The delivery device can be introduced in to a vessel using methods known in the art. Typically, a guidewire is inserted into the vessel using the Seldinger technique and the delivery device is guided over the guidewire to the vessel and region of interest. Once the delivery device 100 is inserted, the operator can obtain real-time images of the luminal surface of the vessel using imaging element. Using the real-time imaging of the luminal surface of the vessel 185, the operator is able to locate a target implantation site, such as target implantation site 190. In certain embodiments, the one or more sensors 12 are used to measure functional flow parameters within the vessel to assist in identifying an implantation site. For example, the one or more sensors 12 can indicate areas of low or high blood pressure within a vessel.

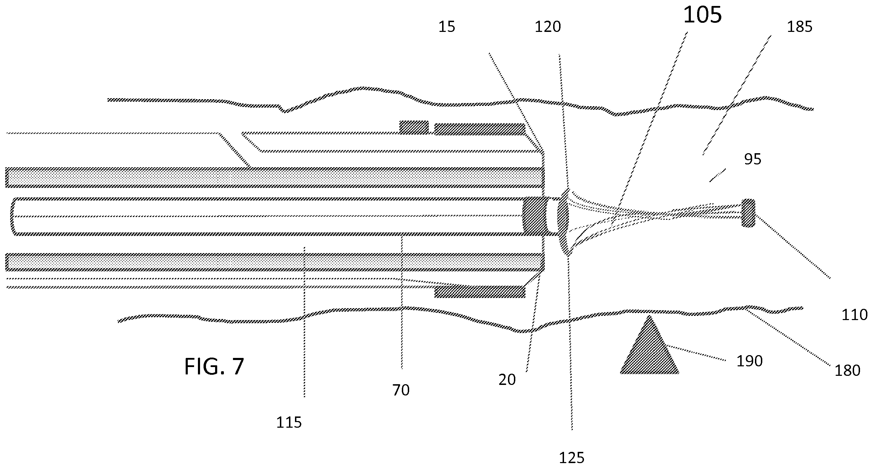

After the target implantation site 190 is located, the operator places the distal tip 15 of the delivery device proximal to the target implantation site 190 (as shown in FIG. 7). A user interface module (included in operating systems 40) shown in FIG. 1) connected to the imaging element 10 and the elongate body 25 can assist the operator in determining the amount of pull back of the elongate body 25 required so that the distal tip 15 is located proximal to the implantation site 190 located by the imaging element 10.

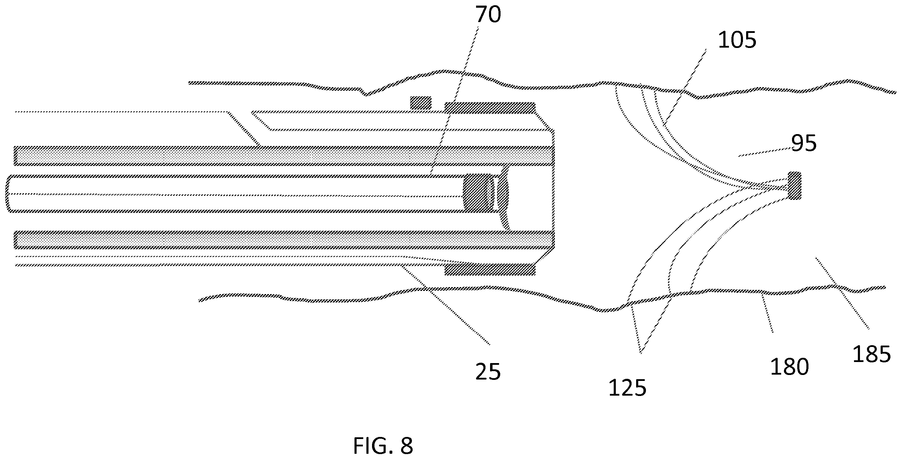

Once the elongate body 25 is positioned for deployment, the inner member 70 is moved distally through the center lumen 115 of the elongate body 25 to push the filter 95 out of the opening 20 of the distal tip 15. As shown, the push member 120 of the inner member 70 engages with the filter legs 105 to deploy the filter 95 into the vessel lumen 185 towards the implantation site 190. Once the proximal ends 125 of the filter legs 105 exit into the vessel lumen 185 from the opening 20, the legs 105 spring open and attach themselves via the hook ends to the vessel wall 180. To assist with expansion of the filter legs 105, the inner member 70 can be retracted back into the center lumen 115 and away from the filter legs 105. FIG. 8 shows the filter 95 as implanted within the vessel 180 with the inner member 80 retracted back into the elongate body 25.

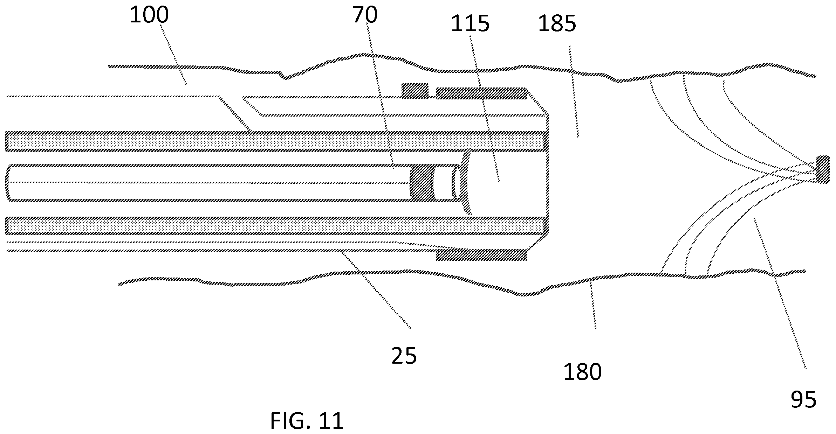

When the filter is placed in the vessel, the inner member 70 can be deployed out of the opening 20 and into the vessel lumen 185 to provide real-time images of the filter 95 as engaged with the vessel wall 180. As shown in FIG. 9, the inner member 70 is deployed into the opening and into the funnel-like cavity 30 formed between the plurality of filter legs 95. The imaging element 90 of the inner member 70 can obtain real-time images to evaluate and ensure that the filter leg hook ends 125 of the filter 95 are properly attached to the wall of the vessel 180. FIG. 10 shows a cross-section of the vessel 180 with the imaging element 90 of the inner member 70 disposed between the hook ends 125 of filter legs 105 engaged with the wall of the vessel 180. After visual confirmation of the implanted filter 95, the inner member 70 is retracted back into the center lumen 115 of the elongate body 25, and the delivery device 100 can be removed from the vessel lumen 185 (as shown in FIG. 11). In certain embodiments, the one or more sensors 12 are used to assess functional flow parameters within the vessel in order to assess the success of the implantation, e.g. whether blood is flowing through the implant properly.

FIGS. 14A-14C illustrate another embodiment of the delivery device 100. In this embodiment, the delivery device 100 is a duel sheath system (that delivers an implant using an outer sheath 1102 and an inner sheath 1103). The delivery device 100 of FIGS. 14A-14C may be used to delivery any type of implant, such as a filter, valve, etc.

As shown in FIG. 14A, the delivery device 100 includes an outer sheath 1102 that is coupled to a connector fitting 1135 (similar to connector fitting 35) at a proximal end. The outer sheath 1102 is an elongate body (such as elongate body 25) that defines a lumen configured to receive an inner delivery sheath 1103 (as shown in FIGS. 14A-14C). The connector fitting 1135 allows transmission lines of an imaging element 10 and/or one or more sensors 12 to connect to an operating system 40 (such as shown in FIG. 2). The outer sheath 1102 may include an imaging element 10 and one or more sensors 12 at a distal portion of the outer sheath 1102. The imaging element 10 and the one or more sensors 12 may have the same configuration on the outer sheath 1102 as it does on the elongate body 25 of the embodiment depicted in FIGS. 1-3. The distal end of the outer sheath 1102 defines an opening 1150 through which an implant 1104 may be delivered.

The outer sheath 1102, like the elongate body 25, may include one or more radiopaque markers 1128. The radiopaque markers 28, when used in conjunction with an external imaging modality such as an angiogram, indicate the positioning of the catheter disposed within a subject. The radiopaque markers 28 may be formed from a radiopaque object or a radiopaque ink. In certain embodiments, the radiopaque markers 28 are spaced a certain distance apart in order to allow easier assessment as to the relative position of the elongate body 25 within the vasculature. The spatially-separated radiopaque markers 28 may indicate when the device is a certain distance within a particular vessel of interest. For example, the radiopaque markers 28 may indicate when the device is 25 cm inside the femoral vein, which is an ideal distance within the vein to place an implant such as a valve stent.

For implant delivery using the device shown in FIGS. 14A-14C, the implant is deployed by translating the outer sheath 1102 with respect to an inner sheath 1103. The inner sheath 1103 for deploying the implant can be introduced into the outer sheath 1102 through a port 1140 of the connector fitting 1135. The outer sheath 1102 may be positioned within the vasculature prior to introduction of the inner sheath 1103 into the outer sheath 1102. In this embodiment, the inner sheath 1103 can be used to drive an implant 1104 through the outer sheath 1102. Alternatively, the inner sheath 1103 may be pre-disposed within a lumen of the outer sheath 1102 and then both sheaths may be introduced concurrently into the vasculature. In this embodiment, an implant may be disposed within a distal portion of the outer sheath 1103 and positioned on or against a distal end of the inner sheath 1103 also disposed within the outer sheath 1103.

The duel-sheath delivery system depicted in FIGS. 14A-14C may be guided to a location of interest within the vasculature using a guidewire. As shown in FIGS. 14A-14C, the system 100 has an over-the-wire configuration, in which the system may be ridden over a guidewire placed in the distal opening 1150 of the outer sheath 1103. The implant 1104 and inner sheath 1103 are likewise configured to receive a guidewire. For example, the implant defines an opening or space through which the guidewire can be retracted past the deployed implant and out of the body.

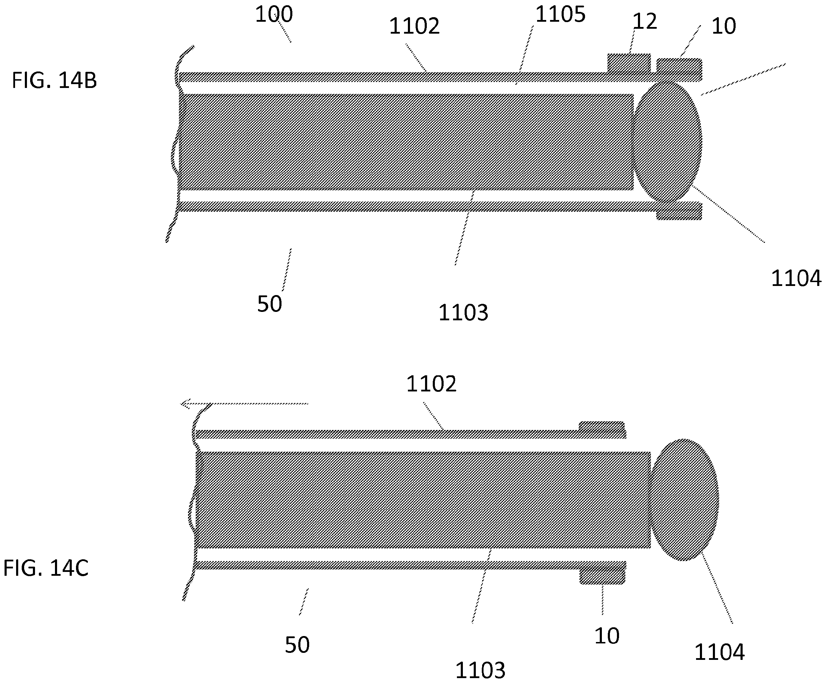

FIGS. 14B and 14C depict deployment of an implant using the duel-sheath delivery device 100. As shown in FIG. 14B, the inner sheath 1103 and implant 1104 disposed within the distal portion 50 of the outer sheath 1102. Prior to deployment, the imaging element 10 and/or the one or more sensors 12 can be used to determine the desired location within the vasculature for implant deployment. The radiopaque markers 1128 may also be used in conjunction with the imaging element 10 and sensors 12 to determine the desired implantation location.

In the un-deployed state (FIG. 14B), an implant 1104 (such as implant 95, 200, 204) is disposed within the lumen 1105 of the outer sheath 1102 and rests against the distal end of the inner sheath 1102. For deployment, the outer sheath 1102 translates proximally with respect to the inner sheath 1103 as shown in FIG. 14C. Due to the proximal translation of the outer sheath 1102, the implant 1104 is deployed through the opening 1150 and into the vessel. Alternatively, the inner sheath 1102 may translate distally relative to the outer sheath 1103 to deploy the implant.

In addition to delivery filter implants as described above, devices of the invention (e.g. depicted in FIGS. 1-3 and 14A-14C) can also be beneficially used to deliver valve stents. Valve stents are often used to replace a diseased valve (e.g. a heart valve) that no longer opens and closes properly. Heart valves help manage blood flow through the heart chamber, the left and right atria and the left and right ventricles, each of which includes its own one-way valve. The natural heart valves are identified as the aortic, mitral (or bicuspid), tricuspid and pulmonary valves. Prosthetic heart valves can be used to replace any of these naturally occurring valves, although repair or replacement of the aortic or mitral valves is most common because they reside in the left side of the heart where pressures are the greatest. Devices of the invention can be used to a) determine whether replacement of a heart valve is necessary (i.e. assess whether the natural valve is diseased or damage), b) delivery an replacement implant valve and c) assess the success and function of the implant valve after implantation.

Diseased or damaged valves may include heart valves that do not open fully due to calcium deposits or scarring, which both attribute to a condition known as stenosis. Valve stenosis is known as an abnormal constriction of a heart valve, which causes less blood to flow through the chamber and onward to the rest of one's body. A damaged valve may also cause a condition known as insufficiency or regurgitation. Valve insufficiency is the leaking of blood through a valve due to an inability of a valve to close tightly. Leakage through a valve generally causes a heart to operate less efficiently, as the heart must work harder to maintain a proper amount of blood flow there through. Valve insufficiency may be caused by torn, loose, or thin valve tissue. Valve insufficiency and stenosis are both associated with abnormal changes in blood flow and pressure within the heart.

An advantage of devices 100 of the invention is that the imaging element 10 and the one or more sensors 12 can be used to indicate whether stenosis or valve insufficiency is present. For example, calcium deposits and scarring that lead to stenosis can be visually indicated, and changes in blood pressure and flow can be assessed using the one or more sensors. If there is abnormal blood flow or pressure, the valve may suffer from stenosis or have damaged tissue. In some circumstances, lower than normal blood flow and pressure is associated with stenosis and/or valve insufficiency. Pressure and flow sensors 12 suitable for use with devices of the invention to determine whether blood flow through a valve is abnormal are described in more detail hereinafter. In addition, imaging elements 10 suitable for use with devices of the invention to assess diseased or damaged valves are described in more detail hereinafter.

Replacement valves for implantation are known in the art and come in several different varieties. Suitable replacement valves that can be implanted using devices of the invention include, for example, replacement valves with an expandable frame and one or more valve leaflets disposed within the expandable frame. Specific examples of valves that may be implanted using devices of the invention include, for example, the Lotus valve (Boston Scientific Inc., MN USA), Centera valve (Edward Lifesciences Inc., CA USA), Portico valve (St. Jude Medical Inc., MN, USA), Acurate Vavle, (Symetis, Ecublens, VD, Switzerland), Engager valve (Medtronic, Inc., USA), and the JenaClip (JenaValve, Inc., Munich Germany). In addition, the following U.S. Patents and Publications describe valves suitable for use with devices of the invention: 8,500,798; 8,454,685; 8,353,954; 8,454,686; 8,597,349; 8,349,000; 2013/0338766; 2013/0053949; and 2013/0218267.

In certain embodiments, in addition to a filter or a valve, the delivery devices of the invention may also be used to insert one or more sensors within the vessel. Preferably, the sensors are biodegradable. The sensors may be separate from the filter or the valve or a part of the filter or the valve. Suitable sensors include pressure sensors, flow sensors, pH sensors, temperature sensors, etc.

Catheter bodies intended for intravascular introduction(such as the elongate body, inner member, inner sheath, and outer sheath)of the delivery device, will typically have a length in the range from 50 cm to 200 cm and an outer diameter in the range from 1 French to 12 French (0.33 mm: 1 French), usually from 3 French to 9 French. In the case of coronary catheters, the length is typically in the range from 125 cm to 200 cm, the diameter is preferably below 8 French, more preferably below 7 French, and most preferably in the range from 2 French to 7 French. Catheter bodies will typically be composed of an organic polymer that is fabricated by conventional extrusion techniques. Suitable polymers include polyvinylchloride, polyurethanes, polyesters, polytetrafluoroethylenes (PTFE), silicone rubbers, natural rubbers, and the like. Optionally, the catheter body may be reinforced with braid, helical wires, coils, axial filaments, or the like, in order to increase rotational strength, column strength, toughness, pushability, and the like. Suitable catheter bodies may be formed by extrusion, with one or more channels being provided when desired. The catheter diameter can be modified by heat expansion and shrinkage using conventional techniques. The resulting catheters will thus be suitable for introduction to the vascular system, often the coronary arteries, by conventional techniques.

The distal portion of the catheters of the present invention may have a wide variety of forms and structures. In many embodiments, a distal portion of the catheter is more rigid than a proximal portion, but in other embodiments the distal portion may be equally as flexible as the proximal portion. One aspect of the present invention provides catheters having a distal portion with a reduced rigid length. The reduced rigid length can allow the catheters to access and treat tortuous vessels and small diameter body lumens. In most embodiments a rigid distal portion or housing of the catheter body will have a diameter that generally matches the proximal portion of the catheter body, however, in other embodiments, the distal portion may be larger or smaller than the flexible portion of the catheter.

A rigid distal portion of a catheter body can be formed from materials that are rigid or which have very low flexibilities, such as metals, hard plastics, composite materials, NiTi, steel with a coating such as titanium nitride, tantalum, ME-92 (antibacterial coating material), diamonds, or the like. Most usually, the distal end of the catheter body will be formed from stainless steel or platinum/iridium. The length of the rigid distal portion may vary widely, typically being in the range from 5 mm to 35 mm, more usually from 10 mm to 25 mm, and preferably between 6 mm and 8 mm. In contrast, conventional catheters typically have rigid lengths of approximately 16 mm. The opening 1001 of the present invention will typically have a length of approximately 2 mm. In other embodiments, however, the opening can be larger or smaller.

The inner member (i.e. push rod or sheath) disposed within the delivery system can include any suitable material having a shaft with enough rigidity to deploy an implant while being flexible enough to move through a body lumen. Like the catheter, the inner member can be formed from polymers optionally reinforced with braid, helical wires, coils, axial filaments, or the like, in order to increase rotational strength, column strength, toughness, pushability, and the like. Suitable polymers include polyvinylchloride, polyurethanes, polyesters, polytetrafluoroethylenes (PTFE), silicone rubbers, natural rubbers, and the like.

According to all embodiments, the delivery device includes one or more imaging elements. In certain aspects, the elongate body of the delivery device includes an imaging element and the inner member of the delivery device includes an imaging element. The imaging element of the elongate body and the imaging element of the inner member may be the same or different. Imaging elements suitable for use with the delivery devices of the invention are described hereinafter. The imaging element is a component of an imaging assembly. Any imaging assembly may be used with devices and methods of the invention, such as optical-acoustic imaging apparatus, intravascular ultrasound (IVUS) or optical coherence tomography (OCT). The imaging element is used to send and receive signals to and from the imaging surface that form the imaging data.

The imaging assembly may be an intravascular ultrasound (IVUS) imaging assembly. IVUS uses an ultrasound probe attached at the distal end. The ultrasound probe can either be either a rotating transducer or an array of circumferentially positioned transducers. For example and as shown in throughout the figures (e.g. FIG. 3), the ultrasound probe can be the imaging element 10 on the elongate body 25 and/or imaging element 90 on the inner member 70. The proximal end of the catheter is attached to computerized ultrasound equipment. The IVUS imaging element (i.e. ultrasound probe) includes transducers that image the tissue with ultrasound energy (e.g., 20-50 MHz range) and image collectors that collect the returned energy (echo) to create an intravascular image. The imaging transducers and imaging collectors are coupled to signal lines that run through the length of the catheter and couple to the computerized ultrasound equipment. For example, the signal lines 65 and 80 coupled to the imaging elements or sensors shown throughout the Figures, including in FIG. 3.

IVUS imaging assemblies produce ultrasound energy and receive echoes from which real time ultrasound images of a thin section of the blood vessel are produced. The imaging transducers of the imaging element are constructed from piezoelectric components that produce sound energy at 20-50 MHz. The image collectors of the imaging element comprise separate piezoelectric elements that receive the ultrasound energy that is reflected from the vasculature. Alternative embodiments of imaging assembly may use the same piezoelectric components to produce and receive the ultrasonic energy, for example, by using pulsed ultrasound. That is, the imaging transducer and the imaging collectors are the same. Another alternative embodiment may incorporate ultrasound absorbing materials and ultrasound lenses to increase signal to noise.

IVUS data is typically gathered in segments where each segment represents an angular portion of an IVUS image. Thus, it takes a plurality of segments (or a set of IVUS data) to image an entire cross-section of a vascular object. Furthermore, multiple sets of IVUS data are typically gathered from multiple locations within a vascular object (e.g., by moving the transducer linearly through the vessel). These multiple sets of data can then be used to create a plurality of two-dimensional (2D) images or one three-dimensional (3D) image.

IVUS imaging assemblies and processing of IVUS data are described in further detail in, for example, Yock, U.S. Pat. Nos. 4,794,931, 5,000,185, and 5,313,949; Sieben et al., U.S. Pat. Nos. 5,243,988, and 5,353,798; Crowley et al., U.S. Pat. No. 4,951,677; Pomeranz, U.S. Pat. No. 5,095,911, Griffith et al., U.S. Pat. No. 4,841,977, Maroney et al., U.S. Pat. No. 5,373,849, Born et al., U.S. Pat. No. 5,176,141, Lancee et al., U.S. Pat. No. 5,240,003, Lancee et al., U.S. Pat. No. 5,375,602, Gardineer et at., U.S. Pat. No. 5,373,845, Seward et al., Mayo Clinic Proceedings 71(7):629-635 (1996), Packer et al., Cardiostim Conference 833 (1994), "Ultrasound Cardioscopy," Eur. J.C.P.E. 4(2):193 (June 1994), Eberle et al., U.S. Pat. No. 5,453,575, Eberle et al., U.S. Pat. No. 5,368,037, Eberle et at., U.S. Pat. No. 5,183,048, Eberle et al., U.S. Pat. No. 5,167,233, Eberle et at., U.S. Pat. No. 4,917,097, Eberle et at., U.S. Pat. No. 5,135,486, U.S. Pub. 2009/0284332; U.S. Pub. 2009/0195514 A1; U.S. Pub. 2007/0232933; and U.S. Pub. 2005/0249391 and other references well known in the art relating to intraluminal ultrasound devices and modalities.

In other embodiments, the imaging assembly may be an optical coherence tomography imaging assembly. OCT is a medical imaging methodology using a miniaturized near infrared light-emitting probe. As an optical signal acquisition and processing method, it captures micrometer-resolution, three-dimensional images from within optical scattering media (e.g., biological tissue). Recently it has also begun to be used in interventional cardiology to help diagnose coronary artery disease. OCT allows the application of interferometric technology to see from inside, for example, blood vessels, visualizing the endothelium (inner wall) of blood vessels in living individuals.

OCT systems and methods are generally described in Castella et al., U.S. Patent No. 8,108,030, Milner et al., U.S. Patent Application Publication No. 2011/0152771, Condit et al., U.S. Patent Application Publication No. 2010/0220334, Castella et al., U.S. Patent Application Publication No. 2009/0043191, Milner et al., U.S. Patent Application Publication No. 2008/0291463, and Kemp, N., U.S. Patent Application Publication No. 2008/0180683 .

In OCT, a light source delivers a beam of light to an imaging device to image target tissue. Light sources can include pulsating light sources or lasers, continuous wave light sources or lasers, tunable lasers, broadband light source, or multiple tunable laser. Within the light source is an optical amplifier and a tunable filter that allows a user to select a wavelength of light to be amplified. Wavelengths commonly used in medical applications include near-infrared light, for example between about 800 nm and about 1700 nm.

Aspects of the invention may obtain imaging data from an OCT system, including OCT systems that operate in either the time domain or frequency (high definition) domain. Basic differences between time-domain OCT and frequency-domain OCT is that in time-domain OCT, the scanning mechanism is a movable mirror, which is scanned as a function of time during the image acquisition. However, in the frequency-domain OCT, there are no moving parts and the image is scanned as a function of frequency or wavelength.

In time-domain OCT systems an interference spectrum is obtained by moving the scanning mechanism, such as a reference mirror, longitudinally to change the reference path and match multiple optical paths due to reflections within the sample. The signal giving the reflectivity is sampled over time, and light traveling at a specific distance creates interference in the detector. Moving the scanning mechanism laterally (or rotationally) across the sample produces two-dimensional and three-dimensional images.

In frequency domain OCT, a light source capable of emitting a range of optical frequencies excites an interferometer, the interferometer combines the light returned from a sample with a reference beam of light from the same source, and the intensity of the combined light is recorded as a function of optical frequency to form an interference spectrum. A Fourier transform of the interference spectrum provides the reflectance distribution along the depth within the sample.

Several methods of frequency domain OCT are described in the literature. In spectral-domain OCT (SD-OCT), also sometimes called "Spectral Radar" (Optics letters, Vol. 21, No. 14 (1996) 1087-1089), a grating or prism or other means is used to disperse the output of the interferometer into its optical frequency components. The intensities of these separated components are measured using an array of optical detectors, each detector receiving an optical frequency or a fractional range of optical frequencies. The set of measurements from these optical detectors forms an interference spectrum (Smith, L. M. and C. C. Dobson, Applied Optics 28: 3339-3342), wherein the distance to a scatterer is determined by the wavelength dependent fringe spacing within the power spectrum. SD-OCT has enabled the determination of distance and scattering intensity of multiple scatters lying along the illumination axis by analyzing a single the exposure of an array of optical detectors so that no scanning in depth is necessary. Typically the light source emits a broad range of optical frequencies simultaneously.

Alternatively, in swept-source OCT, the interference spectrum is recorded by using a source with adjustable optical frequency, with the optical frequency of the source swept through a range of optical frequencies, and recording the interfered light intensity as a function of time during the sweep. An example of swept-source OCT is described in U.S. Pat. No. 5,321,501.

Generally, time domain systems and frequency domain systems can further vary in type based upon the optical layout of the systems: common beam path systems and differential beam path systems. A common beam path system sends all produced light through a single optical fiber to generate a reference signal and a sample signal whereas a differential beam path system splits the produced light such that a portion of the light is directed to the sample and the other portion is directed to a reference surface. Common beam path systems are described in U.S. Pat. 7,999,938; U.S. Pat. 7,995,210; and U.S. Pat. 7,787,127 and differential beam path systems are described in U.S. Pat. 7,783,337; U.S. Pat. 6,134,003; and U.S. Pat. 6,421,164 .

In yet another embodiment, the imaging assembly is an optical-acoustic imaging apparatus. Optical-acoustic imaging apparatus include at least one imaging element to send and receive imaging signals. In one embodiment, the imaging element includes at least one acoustic-to-optical transducer. In certain embodiments, the acoustic-to-optical transducer is an Fiber Bragg Grating within an optical fiber. In addition, the imaging elements may include the optical fiber with one or more Fiber Bragg Gratings (acoustic-to-optical transducer) and one or more other transducers. The at least one other transducer may be used to generate the acoustic energy for imaging. Acoustic generating transducers can be electric-to-acoustic transducers or optical-to-acoustic transducers. The imaging elements suitable for use in devices of the invention are described in more detail below.

Fiber Bragg Gratings for imaging provides a means for measuring the interference between two paths taken by an optical beam. A partially-reflecting Fiber Bragg Grating is used to split the incident beam of light into two parts, in which one part of the beam travels along a path that is kept constant (constant path) and another part travels a path for detecting a change (change path). The paths are then combined to detect any interferences in the beam. If the paths are identical, then the two paths combine to form the original beam. If the paths are different, then the two parts will add or subtract from each other and form an interference. The Fiber Bragg Grating elements are thus able to sense a change wavelength between the constant path and the change path based on received ultrasound or acoustic energy. The detected optical signal interferences can be used to generate an image using any conventional means.

Exemplary optical-acoustic imaging assemblies are disclosed in more detail in U.S. Patent Nos. 6,659,957 and 7,527,594, 7,245.789, 7447,388, 7,660,492, 8,059,923 and in U.S. Patent Publication Nos. 2008/0119739, 2010/0087732 and 2012/0108943.

According to all embodiments, devices of the invention include more sensors to obtain functional flow measurements within the vessel. The functional flow data can be used to assess whether blood flow through a vessel or a valve is normal. This can then be used to determine whether an interventional procedure is necessary (e.g. introduction of an implant to restore normal blood flow) and/or to verify success of an implant deployed by devices of the invention (e.g. whether an implant restored normal blood flow).

The functional flow data obtained by the one or more sensors may be used to assist in locating a implantation site and to assess the restoration of blood flow after introduction of an implant such as a filter or valve, In such embodiments, the one or more sensors always include a pressure sensor, a flow sensor, and combinations thereof. The pressure sensor and the flow sensor may be fiber optic based. Pressure sensors can be used to measure pressure within a lumen and flow sensors can be used to measure the velocity of blood flow through a lumen. Devices with both a pressure sensor and a flow sensor provides information and data for calculating fractional flow reserve (FFR) using pressure readings, and coronary flow reserve (CFR), or similar, using flow readings.

The ability to measure and compare both the pressure and velocity flow to determine an index of resistance of flow within the vessel allows one to determine an ideal placement for an implant. It has been shown that distal pressure and velocity measurements, particularly regarding the pressure drop-velocity relationship such as Fractional Flow reserve (FFR), Coronary flow reserve (CFR) and combined P-V curves, reveal information about the health of the vessel and its need for an interventional procedure to restore normal functional flow parameters.

A pressure sensor allows one to obtain pressure measurements within a body lumen. A particular benefit of pressure sensors is that pressure sensors allow one to measure of FFR in vessel. FFR is a comparison of the pressure within a vessel at a proximal position and a distal position. The FFR value allows one to assess pressure before and after vascular access creation to determine the impact of the procedure. A pressure sensor can be mounted on the distal portion of delivery device 100 (e.g. FIGS. 1-3 or FIGS, 14A-14C), and can be placed distal or proximal to the imaging sensor. In no embodiments, the pressure sensor can be embedded within the imaging sensor. The pressure sensor can be formed of a crystal semiconductor material having a recess therein and forming a diaphragm bordered by a rim. A reinforcing member is bonded to the crystal and reinforces the rim of the crystal and has a cavity therein underlying the diaphragm and exposed to the diaphragm. A resistor having opposite ends is carried by the crystal and has a portion thereof overlying a portion of the diaphragm. Electrical conductor wires can be connected to opposite ends of the resistor and extend within the elongate body 25 or outer sheath 1103 to the the proximal portion where they connect with the operating system, Additional details of suitable pressure sensors that may be used with devices of the invention are described in U.S. Pat. No. 6,106,476. U.S. Pat. No. 6,106,476 also describes suitable methods for mounting the pressure sensor 104 within a sensor housing.

In all aspects, devices of the invention include a flow sensor. The flow sensor can be used to measure blood flow velocity within the vessel, which can be used to assess coronary flow reserve (CFR), or similar. The flow sensor can be, for example, an ultrasound transducer, a Doppler flow sensor or any other suitable flow sensor, disposed at or in close proximity to the distal end of the elongate body 25 or outer sheath 1102, and can be proximal or distal to the imaging element 10. In no embodiments, the flow sensor is embedded within the imaging element 10. The ultrasound transducer may be any suitable transducer, and may be mounted in the distal end using any conventional method, including the manner described in U.S. Pat. No. 5,125,137, 6,551,250 and 5,873,835.

In some embodiments, a device of the invention includes an imaging and sensor assembly and obtain datasets through the operation of OCT, IVUS, or other imaging hardware associated with the imaging element as well as the functional flow hardware associated with the one or more sensors. In some embodiments, a device of not being part the invention is a computer device such as a laptop, desktop, or tablet computer, and obtains a three-dimensional data set by retrieving it from a tangible storage medium, such as a disk drive on a server using a network or as an email attachment.

Methods not being part of the invention can be performed using software, hardware, firmware, hardwiring, or combinations of any of these. Features implementing functions can also be physically located at various positions, including being distributed such that portions of functions are implemented at different physical locations (e.g., imaging apparatus in one room and host workstation in another, or in separate buildings, for example, with wireless or wired connections).

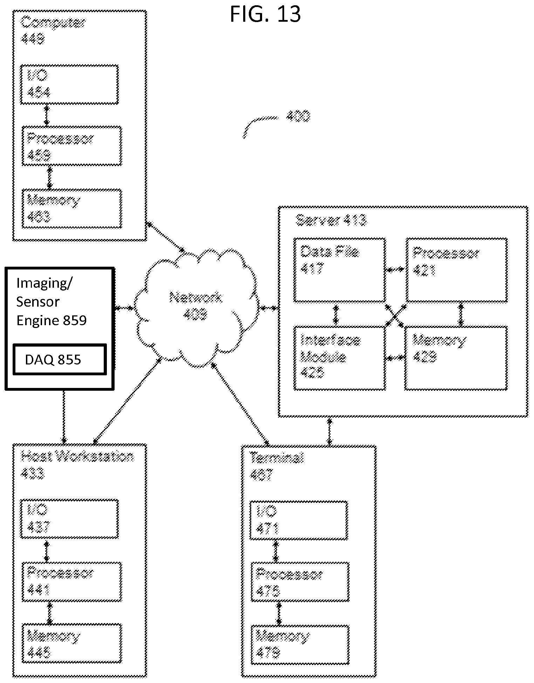

In some embodiments, a user interacts with a visual interface to view images from the imaging system and functional flow data from the one or more sensors. Input from a user (e.g., parameters or a selection) are received by a processor in an electronic device. The selection can be rendered into a visible display. An exemplary system including an electronic device is illustrated in FIG. 13. As shown in FIG. 13, an imaging/sensor engine 859 of the imaging and sensor assemblies communicates with host workstation 433 as well as optionally server 413 over network 409. The data acquisition element 855 (DAQ) of the imaging engine receives imaging data from one or more imaging element. In some embodiments, an operator uses computer 449 or terminal 467 to control system 400 or to receive images and functional flow data. Image and functional flow data may be displayed using an I/O 454, 437, or 471, which may include a monitor. Any I/O may include a keyboard, mouse or touchscreen to communicate with any of processor 421, 459, 441, or 475, for example, to cause data to be stored in any tangible, nontransitory memory 463, 445, 479, or 429. Server 413 generally includes an interface module 425 to effectuate communication over network 409 or write data to data file 417.

Processors suitable for the execution of computer program include, by way of example, both general and special purpose microprocessors, and any one or more processor of any kind of digital computer. Generally, a processor will receive instructions and data from a read-only memory or a random access memory or both. The essential elements of computer are a processor for executing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. Information carriers suitable for embodying computer program instructions and data include all forms of non-volatile memory, including by way of example semiconductor memory devices, (e.g., EPROM, EEPROM, solid state drive (SSD), and flash memory devices); magnetic disks, (e.g., internal hard disks or removable disks); magneto-optical disks; and optical disks (e.g., CD and DVD disks). The processor and the memory can be supplemented by, or incorporated in, special purpose logic circuitry.

To provide for interaction with a user, the subject matter described herein can not be implemented on a computer having an I/O device, e.g., a CRT, LCD, LED, or projection device for displaying information to the user and an input or output device such as a keyboard and a pointing device, (e.g., a mouse or a trackball), by which the user can provide input to the computer. Other kinds of devices can be used to provide for interaction with a user as well. For example, feedback provided to the user can be any form of sensory feedback, (e.g., visual feedback, auditory feedback, or tactile feedback), and input from the user can be received in any form, including acoustic, speech, or tactile input.

The subject matter described herein can not be implemented in a computing system that includes a back-end component (e.g., a data server 413), a middleware component (e.g., an application server), or a front-end component (e.g., a client computer 449 having a graphical user interface 454 or a web browser through which a user can interact with an implementation of the subject matter described herein), or any combination of such back-end, middleware, and front-end components. The components of the system can be interconnected through network 409 by any form or medium of digital data communication, e.g., a communication network. Examples of communication networks include cell network (e.g., 3G or 4G), a local area network (LAN), and a wide area network (WAN), e.g., the Internet.

The subject matter described herein can not be implemented as one or more computer program products, such as one or more computer programs tangibly embodied in an information carrier (e.g., in a non-transitory computer-readable medium) for execution by, or to control the operation of, data processing apparatus (e.g., a programmable processor, a computer, or multiple computers). A computer program (also known as a program, software, software application, app, macro, or code) can be written in any form of programming language, including compiled or interpreted languages (e.g., C, C++, Perl), and it can be deployed in any form, including as a stand-alone program or as a module, component, subroutine, or other unit suitable for use in a computing environment. Systems and methods of the invention can include instructions written in any suitable programming language known in the art, including, without limitation, C, C++, Perl, Java, ActiveX, HTML5, Visual Basic, or JavaScript.

A computer program does not necessarily correspond to a file. A program can be stored in a portion of file 417 that holds other programs or data, in a single file dedicated to the program in question, or in multiple coordinated files (e.g., files that store one or more modules, subprograms, or portions of code). A computer program can be deployed to be executed on one computer or on multiple computers at one site or distributed across multiple sites and interconnected by a communication network.

A file can be a digital file, for example, stored on a hard drive, SSD, CD, or other tangible, non-transitory medium. A file can be sent from one device to another over network 409 (e.g., as packets being sent from a server to a client, for example, through a Network Interface Card, modem, wireless card, or similar).

Writing a file according to the invention involves transforming a tangible, non-transitory computer-readable medium, for example, by adding, removing, or rearranging particles (e.g., with a net charge or dipole moment into patterns of magnetization by read/write heads), the patterns then representing new collocations of information about objective physical phenomena desired by, and useful to, the user. In some embodiments, writing involves a physical transformation of material in tangible, non-transitory computer readable media (e.g., with certain optical properties so that optical read/write devices can then read the new and useful collocation of information, e.g., burning a CD-ROM). In some embodiments, writing a file includes transforming a physical flash memory apparatus such as NAND flash memory device and storing information by transforming physical elements in an array of memory cells made from floating-gate transistors. Methods of writing a file are well-known in the art and, for example, can be invoked manually or automatically by a program or by a save command from software or a write command from a programming language.

### Equivalents

The foregoing embodiments are therefore to be considered in all respects illustrative rather than limiting on the invention described herein. Scope of the invention is thus indicated by the appended claims rather than by the foregoing description, and all changes which come within the meaning of the claims are therefore intended to be embraced therein.

## Claims

1. A system for delivering an implant into the vasculature, the system comprising:
a) a device for delivering an implant into the vasculature comprising:
an outer sheath (25) defining a center lumen (115) that leads to a distal opening, the outer sheath comprising an imaging element (10) configured to at least partially surround the center lumen such that an implant (95) is deployable through the imaging element, wherein the imaging element is positioned on or formed as part of an outer surface of a distal end of the outer catheter sheath;
an inner sheath (70) disposed within the center lumen such that movement of one of the sheaths relative to the other one of the sheaths deploys the implant through the distal opening of the outer sheath;
wherein the outer sheath further comprises a pressure sensor adapted to measure pressure within the vasculature and a flow sensor (12) adapted to measure the velocity of flow through the vasculature, wherein the pressure and flow sensors are located on a distal portion of the outer sheath distal or proximal to the imaging element and are positioned on or formed as part of the outer surface of the outer sheath; and
a connector fitting attached to a proximal portion of the device; and
b) an operating system providing a means to transmit electricity and receive data from the imaging elements and the pressure and flow sensors of the delivery device, wherein through the connector fitting and via a cable the imaging element and the pressure and flow sensors are operably coupled to the operating system,
wherein the system is configured to compare the measured pressure and velocity of flow to determine an index of resistance of flow within the vasculature.

2. The device of claim 1, wherein the outer sheath further comprises one or more radiopaque markers (28) located along a length of the outer sheath.

3. The device of claim 1, wherein the imaging element comprises an ultrasound transducer.

4. The device of claim 1, wherein the device is configured such that a proximal translation of the outer sheath relative to the inner sheath deploys the implant.

5. The device of claim 1, wherein the device is configured such that a distal translation of the inner sheath relative to the outer sheath deploys the implant.

6. The device of claim 1, wherein the implant is selected from the group consisting of a filter, valve, and stent.

7. The device of claim 2, wherein the radiopaque markers are each separated by a distance.

## Patentansprüche

1. Ein System zum Einbringen eines Implantats in das Gefäßsystem, wobei das System Folgendes umfasst:
a) ein Gerät zum Einbringen eines Implantats in das Gefäßsystem, wobei das Gerät Folgendes umfasst:
eine Außenhülle (25), die ein mittiges Lumen (115) definiert, das zu einer distalen Öffnung führt, wobei die Außenhülle ein Bildgebungselement (10) umfasst, das das mittige Lumen zumindest teilweise umgibt, sodass mithilfe des Bildgebungselements ein Implantat (95) eingesetzt werden kann, wobei das Bildgebungselement auf einer Außenfläche des distalen Endes der Katheteraußenhülle positioniert ist oder einen Teil der Hülle bildet;
eine Innenhülle (70) im mittigen Lumen, sodass das Bewegen einer der Hüllen relativ zur anderen das Implantat durch die distale Öffnung der Außenhülle einführt;
wobei die Außenhülle zudem einen Drucksensor zum Messen des Drucks im Gefäßsystem sowie einen Durchflusssensor (12) zum Messen der Flussgeschwindigkeit im Gefäßsystem umfasst, und wobei die Druck- und Durchflusssensoren am distalen Abschnitt der Außenhülle distal oder proximal zum Bildgebungselement sowie an der Außenfläche der Außenhülle positioniert sind oder einen Teil derselben bilden; und
ein Anschlussstück am proximalen Abschnitt des Geräts; und
b) ein Betriebssystem, das eine Vorrichtung zum Übertragen von Elektrizität sowie zum Empfangen von Daten von den Bildgebungselementen und von den Druck-und Durchflusssensoren des Geräts umfasst, wobei das Bildgebungselement und die Druck- und Durchflusssensoren im Betrieb über das Anschlussstück und ein Kabel mit dem Betriebssystem verbunden sind,
wobei das System den gemessenen Druck und die gemessene Flussgeschwindigkeit vergleicht, um einen Index des Strömungswiderstands im Gefäßsystem zu ermitteln.

2. Das Gerät gemäß Anspruch 1, wobei die Außenhülle zudem mindestens eine röntgendichte Markierung (28) umfasst, die entlang der Länge Außenhülle angeordnet ist.

3. Das Gerät gemäß Anspruch 1, wobei das Bildgebungselement einen Ultraschallwandler umfasst.

4. Das Gerät gemäß Anspruch 1, wobei das Gerät so konfiguriert ist, dass eine proximale Verschiebung der Außenhülle relativ zur Innenhülle das Implantat einführt.

5. Das Gerät gemäß Anspruch 1, wobei das Gerät so konfiguriert ist, dass eine distale Verschiebung der Innenhülle relativ zur Außenhülle das Implantat einführt.

6. Das Gerät gemäß Anspruch 1, wobei das Implantat aus einer Gruppe ausgewählt wird, die aus einem Filter, einem Ventil und einem Stent besteht.

7. Das Gerät gemäß Anspruch 2, wobei die röntgendichten Markierungen jeweils um einen Abstand voneinander getrennt sind.

## Revendications

1. Système de placement d'un implant dans le système vasculaire, le système comprenant :
a) un dispositif destiné à placer un implant dans le système vasculaire comprenant :
une gaine extérieure (25) définissant une lumière centrale (115), laquelle guide vers une ouverture distale, la gaine extérieure comprenant un élément d'imagerie (10) conçu pour entourer au moins partiellement la lumière centrale de telle sorte qu'un implant (95) peut être déployé à travers l'élément d'imagerie, dans lequel l'élément d'imagerie est positionné sur ou fait partie d'une surface externe d'une extrémité distale de la gaine de cathéter externe ;
une gaine intérieure (70) disposée à l'intérieur de la lumière centrale de telle sorte que le mouvement de l'une des gaines par rapport à l'autre des gaines déploie l'implant à travers l'ouverture distale de la gaine extérieure ;
dans lequel la gaine externe comprend en outre un capteur de pression conçu pour mesurer la pression à l'intérieur du système vasculaire et un capteur de débit (12) conçu pour mesurer la vitesse d'écoulement à travers le système vasculaire, dans lequel les capteurs de pression et de débit sont situés sur une partie distale de la gaine externe distale ou proximale par rapport à l'élément d'imagerie et sont positionnés sur ou font partie de la surface externe de la gaine externe ; et
un raccord de connecteur fixé à une partie proximale du dispositif ; et
b) un système d'exploitation fournissant un moyen permettant de transmettre l'électricité et de recevoir des données à partir des éléments d'imagerie et des capteurs de pression et de débit du dispositif de placement, dans lequel à travers le raccord de connecteur et par l'intermédiaire d'un câble, l'élément d'imagerie et les capteurs de pression et de débit sont couplés de manière fonctionnelle au système d'exploitation,
dans lequel le système est configuré pour comparer la pression et la vitesse d'écoulement mesurées afin de déterminer un indice de résistance d'écoulement dans le système vasculaire.

2. Dispositif selon la revendication 1, dans lequel la gaine externe comprend en outre au moins un marqueur radio-opaque (28) situé le long d'une longueur de la gaine externe.

3. Dispositif selon la revendication 1, dans lequel l'élément d'imagerie comprend un transducteur à ultrasons.

4. Dispositif selon la revendication 1, dans lequel le dispositif est conçu de telle sorte qu'une translation proximale de la gaine externe par rapport à la gaine interne déploie l'implant.

5. Dispositif selon la revendication 1, dans lequel le dispositif est conçu de telle sorte qu'une translation distale de la gaine interne par rapport à la gaine externe déploie l'implant.

6. Dispositif selon la revendication 1, dans lequel l'implant est choisi dans le groupe constitué par un filtre, une valve et un stent.

7. Dispositif selon la revendication 2, dans lequel les marqueurs radio-opaques sont respectivement séparés par une distance.
